# EUROPEAN PATENT APPLICATION

(11) **EP 2 005 907 A2**
(43) Date of publication of application: **24.12.2008**
(21) Application number: 08011119.8
(22) Date of filing: 19.06.2008
(51) Int. Cl.: A61B 17/34

(54) **Cannula device for endoscopic surgical operations**

(30) Priority: 20.06.2007 IT mi20071230
(71) Applicant: Lucini Surgical Concept S.r.L., 20129 Milano (IT)
(72) Inventor: Lucini, Flavio, Dr. Ing, 20021 Bollate Milano (IT)
(74) Representative: Lunati, Vittoriano

(57) **Abstract**

Cannula device (1) for endoscopic surgical operations comprising: a cylindrical body (2) having an axial direction (2a) and defining a tube (3), an upper base (4) defining an inlet (5) of the tube (3) and a lower base (6) defining an outlet (7) of the tube (3), the inlet (5) and the outlet (7) defining a diagonal (8) joining substantially diametrically opposite points of the inlet (5) and of the outlet (7), wherein the cylindrical body (2) is manually deformable, in a manner suitable to increase the angle (α) of intersection between the diagonal (8) and the axial direction (2a).

## Description

The present invention relates to a cannula device for endoscopic surgical operations, in particular for laparoscopies and thoracoscopies, of the type specified in the preamble of the claim **1**.

Endoscopic surgical operations, or simply endoscopies, are currently well known, characterized by being minimally invasive and performed with specific instruments and tiny video cameras located inside the human body.

During these operations, communication between the inside and the outside of the human body is guaranteed by specific cannulas, called trocars.

Trocars therefore form the channel for the passage of surgical instruments and video cameras and have different functions.

One important function thereof is to prevent continuous contact and rubbing between the tissues of the human body and instruments inserted therein.

Another important function of trocars, in particular trocars for laparoscopy, is to ensure pneumatic seal.

In fact, in laparoscopies the abdominal region is often inflated with specific gases, in order to detach the abdominal wall from the internal organs and obtain improved visibility and increase the space for manoeuvring.

Trocars used for this type of operations therefore ensure pneumatic seal inside the body, by means of specific valves.

Naturally, these valves also allow surgical instruments to be inserted into the trocar.

Trocars are usually inserted by means of a specific sharp pointed tip inserted into the trocar and subsequently removed.

Notwithstanding the advantages of these technologies, which allow operations to be performed through small holes, which do not require large openings to be made in the human body, the aforesaid prior art presents some important drawbacks.

In fact, conventional trocars do not allow the use of standard surgical instruments, which are usually realized by first class levers, i.e. levers with fulcrum interposed between the handle and the active part of the instrument. These instruments are realized, for example, by scissors and forceps.

Moreover, conventional trocars do not allow the surgeon to use his tactile sense. In fact, the surgeon often requires to palpate the inside of the human body for different reasons, in particular to detect the presence of obstructions or irregularities and/or to perform particular surgical manoeuvres, such as sutures.

It is also important to mention that the conventional technique hampers the operation and direct checks by the surgeon also in a manner that is actually dangerous for patients.

In fact, when the trocar is inserted over a said sharp tip, the surgeon must push the trocar-sharp tip system to make it penetrate the tissues until it passes beyond the peritoneum.

In these conditions, it is possible, due to possible excess force, for the sharp tip to reach the intestinal loops and/or important vessels under the peritoneum and not visible to the surgeon. This can obviously cause serious problems for patients.

These drawbacks are worsened by the use of trocars with the described valve, necessary to maintain pressure inside the human body. In fact, these trocars make operations even more complicated and difficult.

This last drawback has been solved by a previous invention by the same applicant, described in the patents IT MI94A1705 and US 5,603,689. This finding allows the abdominal wall to be lifted in laparoscopies without the use of gas, so that the trocar does not require the specific valve or structure.

However, notwithstanding this improvement, the preceding disadvantages of conventional trocars remain unchanged.

In this situation the technical aim of the present invention is to devise a cannula device for endoscopic surgical operations, in particular for laparoscopies and thoracoscopies, capable of substantially overcoming the aforesaid drawbacks.

Within this technical aim, an important object of the invention is to produce a cannula device for endoscopic surgical operations capable of also allowing the surgeon to use conventional surgical instruments.

Another important object of the invention is to produce a cannula device for endoscopic surgical operations capable of allowing the surgeon to palpate the inside of the human body using the fingers and to perform direct manoeuvres on tissues and internal organs, while maintaining the minimum degree of invasiveness.

A further important object of the invention is to facilitate a careful check by the surgeon regarding the presence of any important vessels or the like in the area of perforation of the peritoneum, thereunder, in a manner suitable to prevent any accidental perforation of said vessels or intestinal loops.

The technical aim and the objects specified are achieved by a cannula device for endoscopic surgical operations according to the enclosed Claim 1.

Preferred embodiments are highlighted in the dependent claims.

Further characteristics and advantages of the invention are better clarified below from the detailed description of preferred embodiments of the invention, with reference to the accompanying drawings, wherein:
- **Fig. 1** shows a side view of the device according to the invention;
- **Fig. 2** shows an axonometric view of the device according to the invention;
- **Fig. 3** shows a first example of the upper portion of the device according to the invention;
- **Fig. 4** shows a second example of the upper portion of the device according to the invention;
- **Fig. 5** schematizes, in a bottom view, a particular configuration of the device according to the invention; and
- **Fig. 6** schematizes, in a side view, a particular configuration of the device according to the invention.

With reference to the Figures, the cannula device according to the invention is indicated as a whole with the number **1**.

It is suitable to be secured in a hole made in the human or animal body and in particular in the abdominal wall of the human body.

It comprises, in brief, a cylindrical body **2**, which defines a tube **3**, an upper base **4**, which defines the inlet **5** of the tube 3, and a lower base **6**, which defines the outlet **7** of the tube 3.

The inlet 5 and the outlet 7 define a diagonal **8** joining two substantially diametrically opposite points of the inlet 5 and of the outlet 7.

The cylindrical body 2 is the fundamental portion of the device 1 and has an axial direction **2a**. The outer surface thereof is in fact in close contact with the various tissues of the human body, while the inner surface delimits the tube 3 through which the instruments or video cameras required to perform the endoscopic surgical operation pass.

The cylindrical body 2 is therefore made of a non-toxic and biocompatible material so as not to irritate or infect the portion of tissues with which it comes into contact.

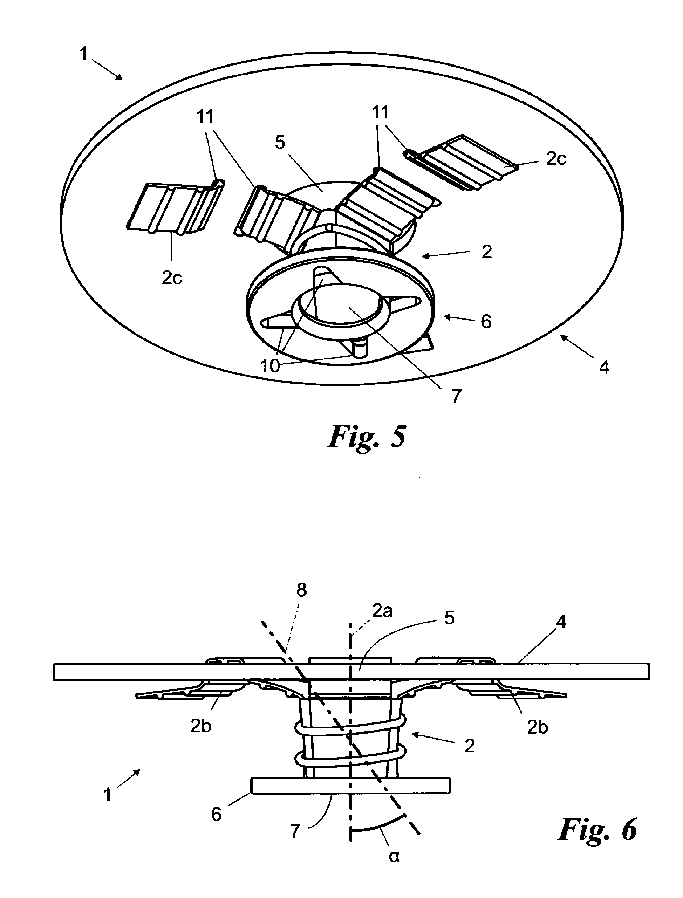

Moreover, the cylindrical body 2 according to the invention is manually deformable, in a manner suitable to increase the angle **α** of intersection between the diagonal 8 and the axial direction 2a, as shown in Figs. 5 and 6.

The cylindrical body 2 is moreover preferably manually deformable substantially in all directions, along the totality of its extension.

Manually obtainable deformability is intended as a flexibility attainable with usual and ordinary efforts that can be made with one hand and this deformability of the cylindrical body 2 is prevalently obtained by means of a specific elastic and flexible polymer material, i.e. a polymer material with low elastic modulus and high strain at yield or break.

The deformability must in fact be such as to be able to be performed manually, i.e. without significant effort.

Different polymer materials can be used for the present purpose, in particular silicones, and among these medical silicone is preferably chosen, for its non-toxic, biocompatible and sterilizable properties.

Moreover, silicones can advantageously be utilized to produced disposable devices 1.

The thickness of the wall of the cylindrical body 2, which defines the deformability of this body 2, can be between 0.1 mm and 4 mm, more preferably between 0.5 mm and 2 mm.

The body 12 also has a geometry that can vary.

In particular, the normal section of the cylindrical body 2 can be elliptical.

Preferably, the greater diameter of the ellipse, when idle, defined by any normal section of the tube 3, is 1.5 times the dimension of the lower diameter. Elliptical geometries having different ratios between major and minor diameter are also included in the inventive concept. These ratios are in any case preferably between 1 (circular section) and 3.

Moreover, the tube 3 can present a normal section that tapers toward one end. It has an average diameter between 15 mm and 27 mm, more preferably between 20 and 22 mm. The height can vary from 30 mm to 100 mm and for example is between 60 and 95 mm.

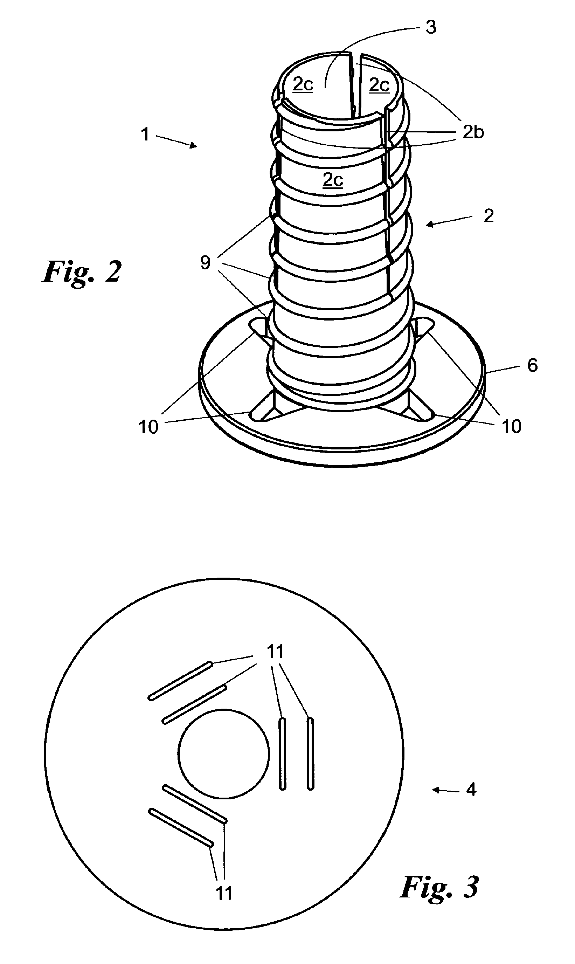

The cylindrical body 2 also comprises, preferably, a plurality of notches **2b**, defining cylindrical sectors **2c** of the cylindrical body 2, flexible in a direction perpendicular to the axial direction 2a, as shown in Figs. 2, 5 and 6.

They are preferably produced starting from one end of the cylindrical body 2 and extend in an axial direction 2a.

Preferably, three notches 2b are present, disposed reciprocally at angular distances of approximately one third of a round angle and having a length greater than two thirds of the cylindrical body 2.

On the outer surface of the cylindrical body 2 there are also preferably present ridges **9** realized by knurls extending on the outer surface of the cylindrical body 2 or by a helicoid.

The ridges 9 preferably have a semicircular section with radius between 0.1 mm and 3 mm, preferably of approximately 1 mm and two consecutive ridges 9 have a reciprocal distance between 4 mm and 12 mm, preferably between 7 mm and 9 mm.

They can also have a triangular arrowhead-shaped section.

The upper base 4 is instead realized substantially by a flattened element and has the function of preventing the device 1 from falling inside the human body and of protecting the outer portion of the human body against rubbing caused by surgical instruments.

It is preferably suitable to secure the cylindrical sectors 2c in a manner suitable to substantially vary the height in axial direction 2a of the cylindrical body 2, in such a manner as to increase the angle α.

The upper base 4 is therefore movable in axial direction with respect to the cylindrical body 2. Moreover, it preferably comprises a plurality of slots **11**, or interlocking means, present in the same or double the number with respect to the number of cylindrical sectors 2c and having dimensions similar to these cylindrical sectors 2c.

These slots 11 thus form retaining elements for the cylindrical sectors 2c, and in particular for the knurls 9, and are suitable to allow the cylindrical sectors 2c to be inserted therein and maintained in the divaricated position, as shown in Figs. 5 and 6.

In particular, if the ridges 9 have triangular sections, these can be inserted and made to slide inside the slots **11** only in one direction and allow safe blocking of the cylindrical sectors 2c in an established position.

They are also useful to allow compression of the tissue in which the device 1 is inserted by means of this device 1 and in particular of the bases **4** and 6. This compression has both haemostatic functions and increases the angle α.

The upper base 4 can be manually elastically deformable.

In this case, it extends over a relatively large area, preferably is substantially ring shaped and has a diameter between 5 cm and 30 cm, as shown in Fig. 3.

Moreover, it is possible for the upper base to have any shape, also suitable to cover the entire human body and if necessary suitable to allow connection to a plurality of cylindrical bodies 2, should the operation require more than one hole to be made in the human body.

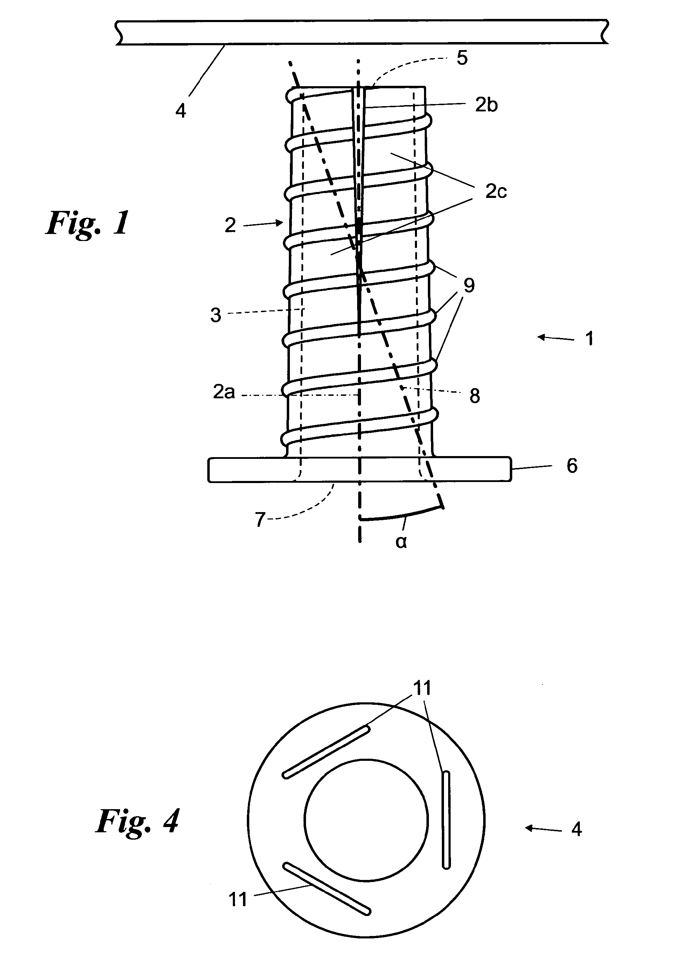

Alternatively, the upper base **4** is realized by a rigid metal element, also ring shaped, as shown in Fig. **4** and having a diameter preferably between 2 cm and 20 cm.

In yet another alternative, the upper base 4 is realized by adhesive elements suitable to constrain, reciprocally or to the body of the patient, the cylindrical sectors 2c and maintain them in a predetermined position.

Finally, the upper base 4 can be provided already joined to the cylindrical body 2; this solution is advisable in particular if the notches 2b are not present.

The lower base 6, which defines the outlet 7 of the tube 3 is instead preferably provided already joined to the cylindrical body 2.

The lower base 6 is realized substantially by a flattened circular ring and is produced in the same polymer material as the cylindrical body 2 and the upper base 4.

The thickness of the lower base 6 can be between 0.1 mm and 5 mm, for example between 1.5 mm and 2.5 mm, while the outer diameter thereof is preferably between 25 mm and 45 mm, for example between 32 mm and 38 mm.

The lower base 6 also presents specific vents **10**, suitable to allow improved deformation in any direction, as shown in Fig. 3. The vents 10 are, for example, substantially triangular in shape, as shown in Fig. 2. The vents 10 are preferably present in the number of four and disposed orthogonal to one another, while the length and height thereof are advantageously between 6 mm and 10 mm. Advantageously, the vents 10 allow improved deformability at least in a direction perpendicular to the axial direction 2a.

Alternatively, the lower base 6 is provided with a plurality of vents 10 in the shape of arc of a circle and present in the number of three, defining substantially three portions of a spiral that facilitate manual insertion and removal of the device 1 in the human body.

Finally, the lower base 6 comprises, a bevel at the join with the tube 3, suitable to facilitate the passage of various instruments and the removal of anatomical portions.

The lower base 6 has the function both of preventing total and accidental release of the device 1 from the human body and, simultaneously, of facilitating the necessary insertion in or removal from the human body of the device 1. It also has the function of compressing the area of the human body above the lower base 6 and, moreover, has a haemostatic function and increases the angle α and thus the manoeuvrability of instruments through the tube 3.

It is in fact possible for losses of blood or other body liquids to occur in this area which prevent or reduce the view of the video cameras. These losses can be reduced or eliminated by compression of the blood vessels by the lower base 6 and facilitate manoeuvrability of the instruments.

Operation of a device 1 for endoscopic surgical operations, in particular for laparoscopies, is as follows.

The device 1 is inserted in the human or animal body by pressing and rotating in the screwing direction of the helicoid 9 in a hole made by a sharp pointed tip or with a conventional scalpel, and/or by retracting forceps. Alternatively, a hole can be made in the human body using a conventional scalpel and the hole can be dilated to a diameter of 20-25 mm using a retractor of known type. There is also an important new technique in which the device according to the invention is inserted over the surgeon's finger and the device is inserted using this finger or, in any case, using a specific instrument having a rounded surface.

After the device 1 has been inserted, the circular sectors 2c are divaricated and the upper base 4 is disposed in such a manner as to constrain them. In particular, the ridges 9 are inserted inside the slots 11, so that they are blocked by the latter.

The device 1 is also preferably disposed in such a manner as to compress the wall of the human body and allow further widening of the angle α.

Alternatively, this operation to insert the upper base can be performed prior to insertion of the device 1.

All these operations can be performed by the surgeon with the aid of forceps, scalpels and various surgical instruments with which the device 1, due to its particular shape and materials, can be deformed.

Consequently, the device 1 remains inside the human body, in particular due to the mechanical block realized by the upper base 4 and by the lower base 6, unless it is forced manually, or in any other manner, in the direction of removal. Also in this case, it is sufficient to pull and rotate the device 1 in the unscrewing direction for it to be rapidly released from the human body.

During the endoscopy the instruments or video cameras required to perform the surgical operation can thus be inserted in the tube 3, without scraping against the body of the patient and causing damage.

Moreover, it is possible to use the device 1 as the outer portion of a trocar of conventional type.

This solution allows use of the device, made of biocompatible and hypoallergenic materials, together with trocars of conventional type.

The invention achieves important advantages.

In particular, the present device 1 allows the use of conventional lever surgical instruments, such as scissors and forceps.

In fact, the deformability of the device 1, in particular of the body 2 and of the upper base 4, allows the angle α of inclination of the diagonal 8 to be increased and therefore the angle for manoeuvring conventional lever instruments can also be increased. In particular, the angle α is increased by decreasing the height of the tube 3, by deforming the section of the device 1 and, moreover, by compressing the wall of the human body.

Moreover, deformability of the cylindrical body 2 allows the surgeon to manoeuvre with greater freedom inside this device 1.

During an endoscopy performed with the device 1 the surgeon can palpate the inside of the human body using a finger.

This prevents serious accidents caused by insertion of a device-sharp tip system not only beyond the peritoneum, but also accidentally or unintentionally reaching intestinal loops or important vessels.

With the structure and the dimensions indicated it is also possible to simultaneously insert more than one surgical instrument into the device, for example a needle holder simultaneously to gripping forceps.

Therefore, there is a considerable increase in the scope of action of surgeons.

## Claims

1. Cannula device (1) for endoscopic surgical operations comprising: a cylindrical body (2) having an axial direction (2a) and defining a tube (3), an upper base (4) defining an inlet (5) of said tube (3) and a lower base (6) defining an outlet (7) of said tube (3), said inlet (5) and said outlet (7) defining a diagonal (8) joining substantially diametrically opposite points of said inlet (5) and said outlet (7), and charaterized in that said cylindrical body (2) is made of a material and having dimensions suitable to allow manual deformation of said cylindrical body (2), in such a manner as to increase the angle (α) of intersection between said diagonal (8) and said axial direction (2a).

2. Device according to Claim 1, wherein said cylindrical body (2) comprises a plurality of notches (2b) defining a plurality of cylindrical sectors (2c) of said cylindrical body (2) flexible in a direction perpendicular to said axial direction (2a).

3. Device according to Claim 2, comprising three notches (2b) disposed reciprocally at angular distances of approximately one third of a round angle.

4. Device according to Claim 2 or 3, wherein said upper base (4) is suitable to secure said cylindrical sectors (2c) in a manner suitable to substantially vary the height in said axial direction (2a) of said cylindrical body (2).

5. Device according to Claim 4, wherein said upper base (4) is movable at least in said axial direction (2a) with respect to said cylindrical body (2).

6. Device according to Claim 5, wherein said upper base (4) comprises a plurality of interlocking means (11), suitable to secure said cylindrical sectors (2c).

7. Device according to one or more of previuous Claims, wherein said upper base (4) is made of flexible material and is suitable to be manually deformed.

8. Device according to one or more of previuous Claims, wherein said upper base (4) is realized by adhesive elements suitable to reciprocally constrain said cylindrical sectors (2c) in a specific position.

9. Device according to one or more of previuous Claims, wherein said upper base (4) is provided already joined to said cylindrical body (2).

10. Device according to Claim 1, presenting ridges (9), disposed on the outer surface of the cylindrical body (2) and suitable to facilitate insertion and removal of the device (1).

11. Device according to Claim 10, wherein said ridges (9) have a triangular arrowhead-shaped section.

12. Device according to one or more of previuous Claims, wherein said tube (3), when not deformed, has a substantially elliptical normal section.

13. Device according to one or more of previuous Claims, wherein said lower base (6) is provided already joined to said cylindrical body (2) and ring shaped projecting from said cylindrical body (2) in said radial direction, and is substantially deformable in all directions and along the entire extension thereof.

14. Device according to one or more of previuous Claims, wherein said cylindrical body (2) is made of silicone.

15. Device according to one or more of previuous Claims, wherein said cylindrical body (2) has walls of a thickness between 0.5 mm and 2 mm.
